# EUROPEAN PATENT SPECIFICATION

(11) **EP 2 571 600 B1**
(45) Date of publication and mention of the grant of the patent: **02.11.2016**
(21) Application number: 11783806.0
(22) Date of filing: 20.05.2011
(51) Int. Cl.: B01D 53/14, B01D 53/72, C02F 3/28, B01D 19/00, B01D 53/78, C12P 7/06, B01D 53/62, B01D 53/96

(54) **ALCOHOL PRODUCTION PROCESS**
VERFAHREN ZUR ALKOHOLHERSTELLUNG
PROCÉDÉ DE PRODUCTION D'UN ALCOOL

(30) Priority: 21.05.2010 US 347327 P
(43) Date of publication of application: 27.03.2013
(73) Proprietor: Lanzatech New Zealand Limited, Auckland 1052 (NZ)
(72) Inventor: COOMBES, Joss, Anton, Auckland 1052 (NZ)
(74) Representative: Keen, Celia Mary
(86) International application number: PCT/NZ2011/000082
(87) International publication number: WO 2011/145956

(56) References cited:
- WO-A1-2009/058028
- WO-A1-2009/058028
- WO-A2-02/08438
- WO-A2-02/08438
- US-A- 4 259 360
- US-A- 6 136 577
- US-A1- 2009 130 734

## Description

### FIELD OF THE INVENTION

This invention relates generally to methods for producing products by anaerobic microbial fermentation of substrates. In particular, the invention relates to methods of increasing efficiency of fermentation by using gas streams exiting a bioreactor to deoxygenate liquid streams entering a bioreactor.

### BACKGROUND OF THE INVENTION

Ethanol is rapidly becoming a major hydrogen-rich liquid transport fuel around the world. Worldwide consumption of ethanol in 2005 was an estimated 12.2 billion gallons. The global market for the fuel ethanol industry has also been predicted to continue to grow sharply in future, due to an increased interest in ethanol in Europe, Japan, the USA and several developing nations.

For example, in the USA, ethanol is used to produce E10, a 10% mixture of ethanol in gasoline. In E10 blends, the ethanol component acts as an oxygenating agent, improving the efficiency of combustion and reducing the production of air pollutants.

In Brazil, ethanol satisfies approximately 30% of the transport fuel demand, as both an oxygenating agent blended in gasoline, and as a pure fuel in its own right. Also, in Europe, environmental concerns surrounding the consequences of Green House Gas (GHG) emissions have been the stimulus for the European Union (EU) to set member nations a mandated target for the consumption of sustainable transport fuels such as biomass derived ethanol.

The vast majority of fuel ethanol is produced via traditional yeast-based fermentation processes that use crop derived carbohydrates, such as sucrose extracted from sugarcane or starch extracted from grain crops, as the main carbon source. However, the cost of these carbohydrate feed stocks is influenced by their value as human food or animal feed, and the cultivation of starch or sucrose-producing crops for ethanol production is not economically sustainable in all geographies. Therefore, it is of interest to develop technologies to convert lower cost and/or more abundant carbon resources into fuel ethanol.

CO is a major, free, energy-rich by-product of the incomplete combustion of organic materials such as coal or oil and oil derived products. For example, the steel industry in Australia is reported to produce and release into the atmosphere over 500,000 tonnes of CO annually.

Catalytic processes may be used to convert gases consisting primarily of CO and/or CO and hydrogen (H₂) into a variety of fuels and chemicals. Micro-organisms may also be used to convert these gases into fuels and chemicals. These biological processes, although generally slower than chemical reactions, have several advantages over catalytic processes, including higher specificity, higher yields, lower energy costs and greater resistance to poisoning.

The ability of micro-organisms to grow on CO as a sole carbon source was first discovered in 1903. This was later determined to be a property of organisms that use the acetyl coenzyme A (acetyl CoA) biochemical pathway of autotrophic growth (also known as the Woods-Ljungdah) pathway and the carbon monoxide dehydrogenase / acetyl CoA synthase (CODH/ACS) pathway). A large number of anaerobic organisms including carboxydotrophic, photosynthetic, methanogenic and acetogenic organisms have been shown to metabolize CO to various end products, namely CO₂, H₂, methane, n-butanol, acetate and ethanol. While using CO as the sole carbon source, all such organisms produce at least two of these end products.

Anaerobic bacteria, such as those from the genus *Clostridium,* have been demonstrated to produce ethanol from CO, CO₂ and H₂ via the acetyl CoA biochemical pathway. For example, various strains of *Clostridium Ijungdahlii* that produce ethanol from gases are described in WO 00/68407, EP 117309, US patent nos. 5,173,429, 5,593,886, and 6,368,819, WO 98/00558 and WO 02/08438. The bacterium *Clostridium autoethanogenum sp* is also known to produce ethanol from gases (Abrini et al., Archives of Microbiology 161, pp 345-351 (1994)).

However, ethanol production by micro-organisms by fermentation of gases is always associated with co-production of acetate and/or acetic acid. As some of the available carbon is converted into acetate/acetic acid rather than ethanol, the efficiency of production of ethanol using such fermentation processes may be less than desirable. Also, unless the acetate/acetic acid by-product can be used for some other purpose, it may pose a waste disposal problem. Acetate/acetic acid is converted to methane by micro-organisms and therefore has the potential to contribute to GHG emissions.

Several enzymes known to be associated with the ability of micro-organisms to use carbon monoxide as their sole source of carbon and energy are known to require metal co-factors for their activity. Examples of key enzymes requiring metal cofactor binding for activity include carbon monoxide dehydrogenase (CODH), and acetyl -CoA synthase (ACS).

WO2007/117157, WO2008/115080, WO2009/022925, WO2009/058028, WO2009/064200, WO2009/064201 and WO2009/113878 describe processes that produce alcohols, particularly ethanol, by anaerobic fermentation of gases containing carbon monoxide. Acetate produced as a by-product of the fermentation process described in WO2007/117157 is converted into hydrogen gas and carbon dioxide gas, either or both of which may be used in the anaerobic fermentation process. WO2009/022925 discloses the effect of pH and ORP In the conversion of substrates comprising CO to products such as acids and alcohols by fermentation. WO2009/058028 describes the use of industrial waste gases for the production of products, such as alcohol, by fermentation. WO2009/064201 discloses carriers for CO and the use of CO in fermentation. WO2009/113878 discloses the conversion of acid(s) to alcohol(s) during fermentation of a substrate comprising CO.

Anaerobic fermentation of substrates comprising CO is typically conducted under strictly anaerobic conditions. Media entering a bloreactor must be substantially deoxygenated prior to passing to the bioreactor to remove substantially all dissolved oxygen. Deoxygenation of the media is a common unit operation requiring energy and additional resources such as an oxygen free gas stream or a vacuum.

Furthermore, gaseous streams exiting the bioreactor typically carry components stripped from the aqueous fermentation broth. Unless volatilised components, such -3-as products including alcohols, can be recovered from the stream exiting the bioreactor, they could be lost in a waste stream.

It is an object of the present invention to provide a process that goes at least some way towards overcoming the above disadvantages, or at least to provide the public with a useful choice.

### SUMMARY OF THE INVENTION

The method of the invention may involve a deoxygenating means comprising a vessel configured for gas/liquid contact comprising:
(i) a first inlet configured to receive liquid;
(ii) a first outlet configured to pass at least a portion of the liquid to a bioreactor;
(iii) a second inlet configured to receive a gaseous stream from a bioreactor; and
(iv) a second outlet configured to pass at least a portion of the gaseous stream away from the vessel.

In particular embodiments, the deoxygenating means is configured such that in use, the liquid and gas flow through the vessel counter-currently.

In particular embodiments, the vessel includes packing material configured to increase the gas/liquid contact surface area. In particular embodiments, the vessel is packed with random or structured packing.

The method of the invention is a method of deoxygenating a liquid nutrient media prior to passing the liquid nutrient media to a bioreactor for anaerobic fermentation, the method including contacting the liquid nutrient media with a gaseous stream exiting the bioreactor as defined in the claims.

In particular embodiments, the liquid nutrient media and the gas stream are contacted in a deoxygenating means configured for effective gas/liquid contact.

In particular embodiments, the method includes fermenting a substrate in the bioreactor to produce one or more products. In particular embodiments, the substrate is gaseous. In particular embodiments, the substrate comprises CO.

In particular embodiments, one or more components of the gaseous stream exiting the bioreactor are captured in the liquid nutrient media when the gaseous stream and liquid nutrient media are contacted.

Thus, the present invention provides a method of improving the efficiency of an anaerobic fermentation, the method including;
a. In a bioreactor containing one or more microorganisms, fermenting one or more gaseous substrates to produce one or more products and a gaseous exit stream;
b. flowing a liquid media broth into a deoxygenating means;
c. passing the gaseous exit stream from the bioreactor to the deoxygenating means;
d. contacting the liquid media broth with an unfermented gaseous exit stream such that one or more components of the gaseous exit stream are captured in the liquid media broth to provide a substantially deoxygenated liquid media broth; and
e. passing the substantially deoxygenated liquid media broth to the bioreactor.

In particular embodiments, the gaseous stream is contacted with an aqueous liquid in deoxygenating means. In particular embodiments, the aqueous liquid is liquid nutrient media fed to the bioreactor to sustain fermentation. In particular embodiments, contacting the gaseous stream with the aqueous liquid substantially deoxygenates he aqueous liquid.

In particular embodiments, the one or more substrates are gaseous. In particular embodiments, the substrate(s) comprise CO.

In particular embodiments, the one or more products include acid(s) and/or alcohol(s). In particular embodiments, the acid(s) include acetate and the alcohol(s) include ethanol.

In particular embodiments, the gaseous stream exiting the bioreactor is passed directly or indirectly to the deoxygenating means, wherein the stream is contacted with the liquid nutrient means. In particular embodiments, the gaseous stream is passed to the deoxygenating means via conduit means.

In particular embodiments, the gaseous stream comprises one or more components including but not limited to gaseous substrate(s) unconverted in the fermentation; gaseous or volatilised product(s) or by-product(s) of fermentation and/or inert gaseous compounds. In particular embodiments, the gaseous stream comprises one or more of CO, CO2, H2, H2S, N2, CH4, alcohols such as ethanol and/or acids such as acetate.

In particular embodiments, the deoxygenating means is as defined above.

The present invention also provides a method for improving the efficiency of a fermentation, the method comprising;
a. in a bioreactor comprising one or more microorganisms, fermenting a gaseous substrate comprising CO to produce one or more liquid products and a gaseous exit stream comprising at least ethanol;
b. passing the gaseous exit stream comprising at least ethanol to a deoxygenating means containing a liquid nutrient broth;
c. contacting the gaseous exit stream comprising at least ethanol with the liquid media broth to provide a deoxygenated liquid media broth;
d. capturing at least a portion of the ethanol component of the gaseous exit stream in the deoxygenated liquid media broth; and
e. passing the deoxygenated liquid nutrient media to the bioreactor for fermentation.

Also provided is a system comprising a bioreactor and deoxygenating means, wherein the deoxygenating means is configured to:
(i) contact a gaseous stream received from the bioreactor with a liquid; and
(ii) pass the substantially deoxygenated liquid to the bioreactor.

In particular embodiments, the deoxygenating means is the deoxygenating means as defined above.

In particular embodiments, the system includes conduit means configured to pass the gaseous stream from the bioreactor to the deoxygenating means. In particular embodiments, the system includes conduit means configured to pass liquid from the deoxygenating means to the bioreactor.

Those skilled in the art will appreciate means for transferring the liquid streams to and from the deoxygenating vessel and the bioreactor. However, by way of example, liquid may be passed to the bioreactor using one or more pumps. Additionally or alternatively, the gaseous stream may be passed to the deoxygenating means using one or more blowers, compressors, fans and/or pumps.

Embodiments of the invention find particular application in the production of acids and alcohols, particularly ethanol by fermentation of a gaseous substrate comprising CO. The substrate may comprise a gas obtained as a by-product of an industrial process. In certain embodiments, the industrial process is selected from the group consisting of ferrous metal products manufacturing, non-ferrous products manufacturing, petroleum refining processes, gasification of biomass, gasification of coal, electric power production, carbon black production, ammonia production, methanol production and coke manufacturing. In one embodiment of the invention, the gaseous substrate is syngas. In one embodiment, the gaseous substrate comprises a gas obtained from a steel mill.

In particular embodiments, the CO-containing substrate will typically contain a major proportion of CO, such as at least about 20% to about 100% CO by volume, from 30% to 70% CO by volume, from 40% to 60% CO by volume, and from 45% to 55% CO by volume. In particular embodiments, the substrate comprises about 25%, or about 30%, or about 35%, or about 40%, or about 45%, or about 50% CO, or about 55% CO, or about 60% CO by volume. In some embodiments, the substrate stream comprises low concentrations of H2, for example, less than 5%, or less than 4%, or less than 3%, or less than 2%, or less than 1%, or is substantially hydrogen free. Substrates having lower concentrations of CO, such as 6%, may also be appropriate, particularly when H₂ and CO₂ are also present.

In various embodiments, the fermentation is carried out using a culture of one or more strains of carboxydotrophic bacteria. In various embodiments, the carboxydotrophic bacterium is selected from *Clostridium, Moorella, Oxobacter, Pepcostreptococcus, Acetobacterium, Eubacterium* or *Butyribacterium.* In one embodiment, the carboxydotrophic bacterium is *Clostridium autoethanogenum*.

### DETAILED DESCRIPTION OF THE INVENTION

It has been surprisingly recognised that a substantially oxygen free outlet gas stream exiting a bioreactor configured for anaerobic fermentation of gaseous substrates, can be used to deoxygenate liquid nutrient media supplied to the bioreactor. This process also has the surprising advantage of substantially capturing at least a portion of one or more products and/or other components from the exiting gas stream in the liquid nutrient media supplied to the bioreactor. The one or more products and/or other products can include alcohols and or acids stripped from an anaerobic fermentation broth in a gaseous outlet stream during fermentation of gaseous substrate. Thus, the invention provides methods and systems of increasing efficiency of capturing one or more products produced by fermentation of a gaseous substrate in a bioreactor.

In particular embodiments, a method of the invention includes the step of passing at least a portion of an outlet gas exiting a bioreactor through media deoxygenating means, such that at least a portion of one or more components in the outlet gas is captured in an aqueous media prior to passing to the bioreactor as defined in the claims. In particular embodiments the one or more components in the outlet gas comprises at least ethanol, and at least a portion of the ethanol is captured in the aqueous media prior to passing to the bioreactor.

Fermentation of gaseous substrates, such as substrates comprising CO, to produce products are known. In such fermentations, a substrate stream is introduced into a bioreactor through one or more conduit means and mixed with a fermentation broth. During fermentation of substrates in a bioreactor, such as substrates comprising CO, one or more microorganisms are typically suspended in a fermentation broth comprising a liquid nutrient media containing nutrients essential for growth and metabolism. Such essential nutrients include, but are not limited to nitrogen, phosphorus, potassium, sulfur and selected B-vitamins, which are typically provided as salts dissolved in an aqueous media. The liquid nutrient media can be provided to a bioreactor containing one or more microorganisms continuously or in batches; wherein the microbial culture converts at least a portion of a substrate, such as a substrate comprising CO, into products, such as alcohol. Fermentation processes, particularly anaerobic fermentation processes, typically include one or more unit operations configured to remove dissolved gases, such as 02, from media provided to the bioreactor.

During fermentation of gaseous substrates, the substrate is typically provided such that at least a portion of the substrate is transferred into solution wherein the microorganisms can metabolise the substrate. For example, a gaseous substrate can be sparged into a fermentation broth at atmospheric or elevated pressure. In accordance with the invention, at least a portion of the substrate is converted to one or more products by fermentation. However, in particular embodiments, a portion of gaseous substrates can be unconsumed in the fermentation and passes through, exiting the bioreactor in an outlet stream. Additionally, or alternatively, gaseous fermentation by-products and/or inert components of the substrate stream will also pass through the bioreactor and exit in an outlet stream.

Thus, in accordance with the invention, a substrate stream is contacted with a fermentation broth in a bioreactor and one or more gaseous components, selected from unfermented gaseous substrate, gaseous fermentation by-product and/or inert gaseous substrate stream components, disengage from the fermentation broth and exit the bioreactor via an outlet conduit. As the gaseous components disengage the fermentation broth, they can volatilise one or more dissolved products, such as alcohols, and carry them out of the bioreactor in the outlet stream. In accordance with the invention, at least a portion of the one or more products can be recovered from a gaseous outlet stream by contacting the outlet stream with media in a deoxygenating means prior to passing the media to the bioreactor.

### Definitions

Unless otherwise defined, the following terms as used throughout this specification are defined as follows:
The term "substrate comprising carbon monoxide" and like terms should be understood to include any substrate in which carbon monoxide is available to one or more strains of bacteria for growth and/or fermentation, for example.

"Gaseous substrate comprising carbon monoxide" include any gas which contains carbon monoxide. The gaseous substrate will typically contain a significant proportion of CO, preferably at least about 5% to about 100% CO by volume.

In the context of fermentation products, the term "acid" as used herein includes both carboxylic acids and the associated carboxylate anion, such as the mixture of free acetic acid and acetate present in a fermentation broth as described herein. The ratio of molecular acid to carboxylate in the fermentation broth is dependent upon the pH of the system. The term "acetate" includes both acetate salt alone and a mixture of molecular or free acetic acid and acetate salt, such as the mixture of acetate salt and free acetic acid present in a fermentation broth as may be described herein. The ratio of molecular acetic acid to acetate in the fermentation broth is dependent upon the pH of the system.

The term "bioreactor" includes a fermentation device consisting of one or more vessels and/or towers or piping arrangements, which includes the Continuous Stirred Tank Reactor (CSTR), Immobilized Cell Reactor (ICR), Trickle Bed Reactor (TBR), Moving Bed Biofilm Reactor (MBBR), Bubble Column, Gas Lift Fermenter, Membrane Reactor such as Hollow Fibre Membrane Bioreactor (HFMBR), Static Mixer, or other vessel or other device suitable for gas-liquid contact.

Unless the context requires otherwise, the phrases "fermenting", "fermentation process" or "fermentation reaction" and the like, as used herein, are intended to encompass both the growth phase and product biosynthesis phase of the process. As will be described further herein, in some embodiments the bioreactor may comprise a first growth reactor and a second fermentation reactor. As such, the addition of metals or compositions to a fermentation reaction should be understood to include addition to either or both of these reactors.

While the following description focuses on particular embodiments of the invention, namely the production of ethanol and/or acetate using CO as the primary substrate, it should be appreciated that the invention may be applicable to production of alternative alcohols and/or acids and the use of alternative substrates as will be known by persons of ordinary skill in the art to which the invention relates. For example, gaseous substrates containing carbon dioxide and hydrogen may be used. Further, the invention may be applicable to fermentation to produce butyrate, propionate, caproate, ethanol, propanol, and butanol. The methods may also be of use in producing hydrogen. By way of example, these products may be produced by fermentation using microbes from the genus *Moorella, Clostridia, Ruminococcus, Acetobacterium, Eubacterium, Butyribacterium, Oxobacter, Methanosarcina, Methanosarcina,* and *Desulfotomaculum.*

### Fermentation

Certain embodiments of the Invention are adapted to use gas streams produced by one or more industrial processes. Such processes include steel making processes, particularly processes which produce a gas stream having a high CO content or a CO content above a predetermined level (i.e., 5%). According to such embodiments, acetogenic bacteria are preferably used to produce acids and/or alcohols, particularly ethanol or butanol, within one or more bioreactors. Those skilled in the art will be aware upon consideration of the instant disclosure that the invention may be applied to various industries or waste gas streams, including those of vehicles with an internal combustion engine. Also, those skilled in the art will be aware upon consideration of the instant disclosure that the invention may be applied to other fermentation reactions including those using the same or different micro-organisms. It is therefore intended that the scope of the invention is not limited to the particular embodiments and/or applications described but is instead to be understood in a broader sense; for example, the source of the gas stream is not limiting, other than that at least a component thereof is usable to feed a fermentation reaction. The invention has particular applicability to improving the overall carbon capture and/or production of ethanol and other alcohols from gaseous substrates comprising CO. Processes for the production of ethanol and other alcohols from gaseous substrates are known. Exemplary processes include those described for example in WO2007/117157, WO2008/115080, WO2009/022925, WO2009/064200, US 6,340,581, US 6,136,577, US 5,593,886, US 5,807,722 and US 5,821,111, each of which is incorporated herein by reference.

A number of anaerobic bacteria are known to be capable of carrying out the fermentation of CO to alcohols, including *n*-butanol and ethanol, and acetic acid, and are suitable for use in the process of the present invention. Examples of such bacteria that are suitable for use in the invention include those of the genus *Clostridium,* such as strains of *Clostridium Ijungdahlii,* including those described in WO 00/68407, EP 117309, US patent No's 5,173,429, 5,593,886, and 6,368,819, WO 98/00558 and WO 02/08438, *Clostridium carboxydivorans* (Liou et al., International Journal of Systematic and Evolutionary Microbiology 33: pp 2085-2091), *Clostridium ragsdalei* (WO/2008/028055) and *Closcridium aucoethanogenum* (Abrini et al, Archives of Microbiology 161: pp 345-351). Other suitable bacteria include those of the genus *Moorel*/*a,* including *Moorella sp* HUC22-1, (Sakal et al, Biotechnology Letters 29: pp 1607-1612), and those of the genus *Carboxydothermus* (Svetlichny, V.A., Sokolova, T.G. et al (1991), Systematic and Applied Microbiology 14: 254-260). Further examples include *Moorella thermoacetica, Moorella thermoautotrophica, Ruminococcus productus, Acetobacterium woodil, Eubacterium limosum, Butyribacterium methylotrophicum, Oxobacter pfennigli, Methanosarcina barkeri, Methanosarcina acetivorans, Desulfotomaculum kuznetsovii* (Simpa et. al. Critical Reviews in Biotechnology, 2006 Vol. 26. Pp41-65). In addition, it should be understood that other acetogenic anaerobic bacteria may be applicable to the present invention as would be understood by a person of skill in the art. It will also be appreciated that the invention may be applied to a mixed culture of two or more bacteria.

One exemplary micro-organism suitable for use in the present invention is *Clostridium autoethanogenum.* In one embodiment, the *Clostridium autoethanogenum* is a *Clostridium aucoethanogenum* having the identifying characteristics of the strain deposited at the German Resource Centre for Biological Material (DSMZ) under the identifying deposit number 19630. In another embodiment, the *Clostridium outoethanogenum* is a *Clostridium autoethanogenum* having the identifying characteristics of DSMZ deposit number DSMZ 23693.

Culturing of the bacteria used in the methods of the invention may be conducted using any number of processes known in the art for culturing and fermenting substrates using anaerobic bacteria. Exemplary techniques are provided in the "Examples" section below. By way of further example, those processes generally described in the following articles using gaseous substrates for fermentation may be utilised: (i) K. T. Klasson, et al. (1991). Bioreactors for synthesis gas fermentations resources. Conservation and Recycling, 5; 145-165; (ii) K. T. Klasson, et al. (1991). Bioreactor design for synthesis gas fermentations. Fuel. 70. 605-614; (iii) K. T. Klasson, et al. (1992). Bioconversion of synthesis gas into liquid or gaseous fuels. Enzyme and Microbial Technology. 14; 602-608; (iv) J. L. Vega, et al. (1989). Study of Gaseous Substrate Fermentation: Carbon Monoxide Conversion to Acetate. 2. Continuous Culture. Biotech. Bioeng. 34. 6. 785-793; (v) J. L. Vega, et al, (1989). Study of gaseous substrate fermentations: Carbon monoxide conversion to acetate. 1. Batch culture. Biotechnology and Bioengineering. 34. 6. 774-784; (vi) J. L. Vega, et al. (1990). Design of Bioreactors for Coal Synthesis Gas Fermentations. Resources, Conservation and Recycling. 3.149-160.

The fermentation may be carried out in any suitable bioreactors, such as one or more continuous stirred tank reactor (CSTR), immobilised cell reactor(s), a gas-lift reactor(s), bubble column reactor(s) (BCR), membrane reactor(s), such as a Hollow Fibre Membrane Bioreactor (HFMBR) or trickle bed reactor(s) (TBR). Also, in some embodiments of the invention, the bioreactor(s) may comprise a first, growth reactor in which the micro-organisms are cultured, and a second, fermentation reactor, to which fermentation broth from the growth reactor is fed and in which most of the fermentation product (e.g. ethanol and acetate) is produced. In particular embodiments, the second bioreactor Is different to the first bioreactor.

According to various embodiments of the invention, the carbon source for the fermentation reaction is a gaseous substrate containing CO. The substrate may be a CO-containing waste gas obtained as a by-product of an industrial process, or from another source such as from automobile exhaust fumes. In certain embodiments, the industrial process is selected from the group consisting of ferrous metal products manufacturing, such as a steel mill, non-ferrous products manufacturing, petroleum refining processes, gasification of coal, electric power production, carbon black production, ammonia production, methanol production and coke manufacturing. In these embodiments, the CO-containing substrate may be captured from the industrial process before it is emitted into the atmosphere, using any convenient method. Depending on the composition of the CO -containing substrate, it may also be desirable to treat it to remove any undesired impurities, such as dust particles before introducing it to the fermentation. For example, the gaseous substrate may be filtered or scrubbed using known methods.

Alternatively, the CO-containing substrate may be sourced from the gasification of biomass. The process of gasification involves partial combustion of biomass in a restricted supply of air or oxygen. The resultant gas typically comprises mainly CO and H₂, with minimal volumes of CO₂, methane, ethylene and ethane. For example, biomass by-products obtained during the extraction and processing of foodstuffs such as sugar from sugarcane, or starch from maize or grains, or non-food biomass waste generated by the forestry industry may be gasified to produce a CO-containing gas suitable for use in the present invention.

The CO-containing substrate will typically contain a major proportion of CO, such as at least about 20% to about 100% CO by volume, from 40% to 95% CO by volume, from 60% to 90% CO by volume, and from 70% to 90% CO by volume. In particular embodiments, the substrate comprises 25%, or 30%, or 35%, or 40%, or 45%, or 50% CO by volume. Substrates having lower concentrations of CO, such as 6%, may also be appropriate, particularly when H₂ and CO₂ are also present.

While it is not necessary for the substrate to contain any hydrogen, the presence of H₂ should not be detrimental to product formation in accordance with methods of the invention. In particular embodiments, the presence of hydrogen results in an improved overall efficiency of alcohol production. For example, in particular embodiments, the substrate may comprise an approx 2:1, or 1:1, or 1:2 ratio of H2:CO. In other embodiments, the substrate stream comprises low concentrations of H2, for example, less than 5%, or less than 4%, or less than 3%, or less than 2%, or less than 1%, or is substantially hydrogen free. The substrate may also contain some CO₂ for example, such as about 1% to about 80% CO₂ by volume, or 1% to about 30% CO₂ by volume. In particular embodiments, the substrate stream comprises CO2 and no or minimal CO.

Typically, the carbon monoxide will be added to the fermentation, reaction in a gaseous state. However, the methods of the invention are not limited to addition of the substrate in this state. For example, the carbon monoxide can be provided in a liquid. For example, a liquid may be saturated with a carbon monoxide containing gas and that liquid added to the bioreactor. This may be achieved using standard methodology. By way of example a microbubble dispersion generator (Hensirisak et. al. Scale-up of microbubble dispersion generator for aerobic fermentation; Applied Biochemistry and Biotechnology Volume 101, Number 3 / October, 2002) could be used for this purpose.

It will be appreciated that for growth of the bacteria and CO-to-alcohol fermentation to occur, in addition to the CO-containing substrate gas, a suitable liquid nutrient medium will need to be fed to the bioreactor. A nutrient medium will contain vitamins and minerals sufficient to permit growth of the micro-organism used. Anaerobic media suitable for the fermentation of ethanol using CO as the sole carbon source are known in the art. For example, suitable media are described in US patent No's 5,173,429 and 5,593,886 and WO 02/08438, WO2007/117157, WO2008/115080, WO2009/022925, WO2009/058028, WO2009/064200, WO2009/064201 and WO2009/113878, referred to above. The present invention provides a novel media which has increased efficacy in supporting growth of the micro-organisms and/or alcohol production in the fermentation process. This media will be described in more detail hereinafter.

The fermentation should desirably be carried out under appropriate conditions for the desired fermentation to occur (for example microbial growth and/or ethanol production). Reaction conditions that should be considered include pressure, temperature, gas flow rate, liquid flow rate, media pH, media redox potential, agitation rate (if using a continuous stirred tank reactor), inoculum level, maximum gas substrate concentrations to ensure that CO in the liquid phase does not become limiting, and maximum product concentrations to avoid product inhibition. Suitable conditions are described in WO02/08438, WO07/117157, WO08/115080 and WO2009/022925.

The optimum reaction conditions will depend partly on the particular micro-organism used. However, in general, it is preferred that the fermentation be performed at pressure higher than ambient pressure. Operating at increased pressures allows a significant increase in the rate of CO transfer from the gas phase to the liquid phase where it can be taken up by the micro-organism as a carbon source for the production of ethanol. This in turn means that the retention time (defined as the liquid volume in the bioreactor divided by the input gas flow rate) can be reduced when bioreactors are maintained at elevated pressure rather than atmospheric pressure.

Also, since a given CO-to-ethanol conversion rate is in part a function of the substrate retention time, and achieving a desired retention time in turn dictates the required volume of a bioreactor, the use of pressurized systems can greatly reduce the volume of the bioreactor required, and consequently the capital cost of the fermentation equipment. According to examples given in US patent no. 5,593,886, reactor volume can be reduced in linear proportion to increases in reactor operating pressure, i.e. bioreactors operated at 10 atmospheres of pressure need only be one tenth the volume of those operated at 1 atmosphere of pressure.

The benefits of conducting a gas-to-ethanol fermentation at elevated pressures have also been described elsewhere. For example, WO 02/08438 describes gas-to-ethanol fermentations performed under pressures of 30 psig and 75 psig, giving ethanol productivities of 150 g/l/day and 369 g/l/day respectively. However, example fermentations performed using similar media and input gas compositions at atmospheric pressure were found to produce between 10 and 20 times less ethanol per litre per day.

It is also desirable that the rate of introduction of the CO-containing gaseous substrate is such as to ensure that the concentration of CO in the liquid phase does not become limiting. This is because a consequence of CO-limited conditions may be that the ethanol product is consumed by the culture.

### Product recovery

The products of the fermentation reaction can be recovered using known methods. Exemplary methods include those described in WO07/117157, WO08/115080, US 6,340,581, US 6,136,577, US 5,593,886, US 5,807,722 and US 5,821,111. However, briefly and by way of example only ethanol may be recovered from the fermentation broth by methods such as fractional distillation or evaporation, and extractive fermentation.

Distillation of ethanol from a fermentation broth yields an azeotropic mixture of ethanol and water (i.e., 95% ethanol and 5% water). Anhydrous ethanol can subsequently be obtained through the use of molecular sieve ethanol dehydration technology, which is also well known in the art.

Extractive fermentation procedures involve the use of a water-miscible solvent that presents a low toxicity risk to the fermentation organism, to recover the ethanol from the dilute fermentation broth. For example, oleyl alcohol is a solvent that may be used in this type of extraction process. Oleyl alcohol is continuously introduced into a fermenter, whereupon this solvent rises forming a layer at the top of the fermenter which is continuously extracted and fed through a centrifuge. Water and cells are then readily separated from the oleyl alcohol and returned to the fermenter while the ethanol-laden solvent is fed into a flash vaporization unit. Most of the ethanol is vaporized and condensed while the oleyl alcohol is non volatile and is recovered for reuse in the fermentation.

Acetate, which is produced as a by-product in the fermentation reaction, may also be recovered from the fermentation broth using methods known in the art.

For example, an adsorption system involving an activated charcoal filter may be used. In this case, it is preferred that microbial cells are first removed from the fermentation broth using a suitable separation unit. Numerous filtration-based methods of generating a cell free fermentation broth for product recovery are known in the art. The cell free ethanol - and acetate - containing permeate is then passed through a column containing activated charcoal to adsorb the acetate. Acetate in the acid form (acetic acid) rather than the salt (acetate) form is more readily adsorbed by activated charcoal. It is therefore preferred that the pH of the fermentation broth is reduced to less than about 3 before it is passed through the activated charcoal column, to convert the majority of the acetate to the acetic acid form.

Acetic acid adsorbed to the activated charcoal may be recovered by elution using methods known in the art. For example, ethanol may be used to elute the bound acetate. In certain embodiments, ethanol produced by the fermentation process itself may be used to elute the acetate. Because the boiling point of ethanol is 78.8 ºC and that of acetic acid is 107 ºC, ethanol and acetate can readily be separated from each other using a volatility-based method such as distillation.

Other methods for recovering acetate from a fermentation broth are also known in the art and may be used in the processes of the present invention. For example, US patent No's 6,368,819 and 6,753,170 describe a solvent and cosolvent system that can be used for extraction of acetic acid from fermentation broths. As with the example of the oleyl alcohol-based system described for the extractive fermentation of ethanol, the systems described in US patent No's 6,368,819 and 6,753,170 describe a water immiscible solvent/co-solvent that can be mixed with the fermentation broth in either the presence or absence of the fermented micro-organisms in order to extract the acetic acid product. The solvent/co-solvent containing the acetic acid product is then separated from the broth by distillation. A second distillation step may then be used to purify the acetic acid from the solvent/co-solvent system.

The products of the fermentation reaction (for example ethanol and acetate) may be recovered from the fermentation broth by continuously removing a portion of the broth from the fermentation bioreactor, separating microbial cells from the broth (conveniently by filtration), and recovering one or more product from the broth simultaneously or sequentially. In the case of ethanol it may be conveniently recovered by distillation, and acetate may be recovered by adsorption on activated charcoal, using the methods described above. The separated microbial cells are preferably returned to the fermentation bioreactor. The cell free permeate remaining after the ethanol and acetate have been removed may also be returned to a fermentation bioreactor. Additional nutrients (such as B vitamins) may be added to the cell free permeate to replenish the nutrient medium before it is returned to the bioreactor. Also, if the pH of the broth was adjusted as described above to enhance adsorption of acetic acid to the activated charcoal, the pH should be re-adjusted to a similar pH to that of the broth in the fermentation bioreactor, before being returned to the bioreactor.

### Deoxygenation Process

In accordance with the invention, there is provided a method of capturing one or more components stripped from an anaerobic fermentation broth in a gaseous outlet stream during fermentation of gaseous substrates as defined in the claims. In particular embodiments, the method includes passing the gaseous outlet stream through deoxygenating means, wherein one or more components in the gaseous outlet stream are transferred to a liquid media. In particular embodiments, the liquid media is deoxygenated by the gaseous outlet stream prior to passing the liquid media to the bioreactor.

Upon consideration of the instant disclosure, those skilled in the art will appreciate known methods of introducing a gaseous substrate stream into a fermentation broth, such that at least a portion of the substrate stream can be converted to products. For example, a gaseous substrate can be sparged into a fermentation broth in a bioreactor, at atmospheric or elevated pressure. Due to the low solubility of most gaseous substrates, such as substrates comprising CO, a proportion of the substrate may pass through the bioreactor without being metabolised by the microorganisms. The unreacted substrate typically exits the bioreactor in an outlet stream. In particular embodiments, unfermented substrate can be optionally returned to the bioreactor via a recycle loop. However, it is recognised that, even in embodiments including a substrate recycle means, at least a portion of the substrate will exit the bioreactor in an outlet stream.

Furthermore, some fermentation reactions produce gaseous products as a waste by-product, which can exit the bioreactor in the outlet stream. Additionally, inert components of a substrate stream, such as components that are not fermented into products, typically pass through and exit the bioreactor in the outlet stream.

For example, during fermentation of a substrate comprising CO to produce products such as alcohols, at least a portion of the CO may pass through the bioreactor without being fermented into products. Such unconverted substrate exits the bioreactor via an outlet port and is typically vented. Other inert gaseous components of a substrate stream may include but is not limited to N2, CH4, He, Ar, CO2. Furthermore, fermentation of substrates comprising CO typically produces CO2 as a by-product of fermentation.

In addition to the gaseous substrate, other gaseous components may be provided to the fermentation broth as part of the substrate stream or in addition to the substrate stream. For example, H2 can be used as a co-substrate in fermentation, such as fermentation of substrate comprising CO. Additionally, or alternatively, one or more essential nutrients required for microbial growth and/or metabolism may be provided in gaseous form, such as H2S.

In fermentation of gaseous substrates, a substrate stream is contacted with a fermentation broth in a bioreactor and one or more gaseous components, selected from unfermented gaseous substrate, gaseous fermentation by-product and/or inert gaseous substrate stream components, disengage from the fermentation broth and exit the bioreactor via an outlet conduit. As the gaseous components disengage the fermentation broth, they can volatilise one or more dissolved products, such as alcohols, and carry them out of the bioreactor in the outlet stream.

In accordance with the invention, at least a portion of the one or more products entrained in the gaseous outlet stream can be recovered by contacting the gaseous outlet stream with media in one or more deoxygenating means. In known anaerobic fermentations, deoxygenating means are used to remove dissolved gaseous components, in particular 02, from media, prior to passing the media to a bioreactor for fermentation. The media is typically aqueous and can contain one or more of the nutrients required for fermentation. Alternatively, one or more nutrients can be added to the media after deoxygenating.

In particular embodiments of the invention, the fermentation is operated in a continuous or semi continuous mode, wherein deoxygenated media is provided to the bioreactor substantially continuously. Typically, a substantially equal volume of fermentation broth will be removed from the bioreactor, such that the volume of the fermentation broth in the bioreactor remains substantially constant. The fermentation broth exiting the bioreactor will typically contain one or more microorganisms, which can be optionally returned to the bioreactor following a separation step; and one or more products which can be separated in one or more product recovery steps.

Deoxygenation is typically achieved through vacuum stripping, membrane stripping, steam stripping or by the use of chemical oxygen scavengers. Additionally or alternatively, deoxygenation can be achieved through contacting an oxygen free gas, such as N2, with the media in a deoxygenating vessel. However, in accordance with the invention, the substantially 02 free gaseous outlet stream exiting the bioreactor is contacted with the media in the deoxygenating means. In particular embodiments, contacting the gaseous outlet stream with the media in the deoxygenating means results in:
(a) effective removal of dissolved gases, such as 02, from the media; and
(b) capture of at least a portion of the product, such as alcohol, from the outlet stream into the media.

It is also recognised that in accordance with particular embodiments of the invention, at least a portion of additional components of the outlet stream, such as H2S, may also be captured into the media, thus beneficially providing a fermentation broth with said component which may be required for microbial growth and/or metabolism.

Those skilled in the art will be aware of deoxygenating means suitable for use with the invention, upon consideration of the instant disclosure. However, by way of non-limiting example, in particular embodiments, a deoxygenating means comprises a vessel configured for gas/liquid contact. In use, a liquid, such as an aqueous media, is passed through the vessel and a deoxygenating stream typically comprising an 02 free gas, such as N2, is also passed through the vessel. The liquid and deoxygenating stream can be passed through the vessel in the same direction; however, the vessel is typically configured such that the liquid and deoxygenating stream flow counter-currently.

Such vessels are typically packed to increase the gas/liquid contact surface area. The packing in such vessels are well known to those skilled in the art. However, by way of non-limiting example, the packing material maybe random or structured and may comprise one or more stacked plates or trays, sieve plates, Raschig rings or the like or a combination thereof. The nature and density of the packing can be selected depending on the size of the vessel to minimise pressure drop across the vessel and achieve the desired level of deoxygenation.

Thus, in accordance with the invention, the deoxygenating stream comprises the outlet stream from the bioreactor; wherein upon contacting the outlet stream with the media, in the deoxygenating means, at least a portion of one or more products, such as alcohol, entrained in the outlet stream, is captured into the media. The deoxygenated media, containing at least a portion of the product(s) can then be passed to the bioreactor for fermentation.

It has also been surprisingly recognised that using the outlet stream as a deoxygenated stream will result in beneficial heat transfer. For example, media entering the deoxygenating means will typically be at ambient temperature, whereas the gaseous outlet stream will be at or towards the optimum fermentation temperature. In accordance with the invention, contacting the gaseous outlet stream with the media will result in heat transfer such that the temperature of the media is heated or cooled toward the optimum fermentation temperature.

In particular embodiments, the optimum fermentation temperature is 37°C, whereas the temperature of the media will be much lower (less than 25°C). Thus, in accordance with particular embodiments, at least a portion of the thermal energy of the gaseous outlet stream will be used to heat the media towards 37°C. In addition, capturing products, such as alcohols, into the aqueous media (condensation) will also have a heating effect.

In accordance with particular aspects, there is provided a system including a bioreactor and deoxygenating means, wherein the deoxygenating means is configured to receive at least a portion of an outlet stream from the bioreactor and contact the outlet stream with media such that in use:
(a) the media is substantially deoxygenated; and
(b) at least a portion of one or more products in the outlet stream are captured in the media.

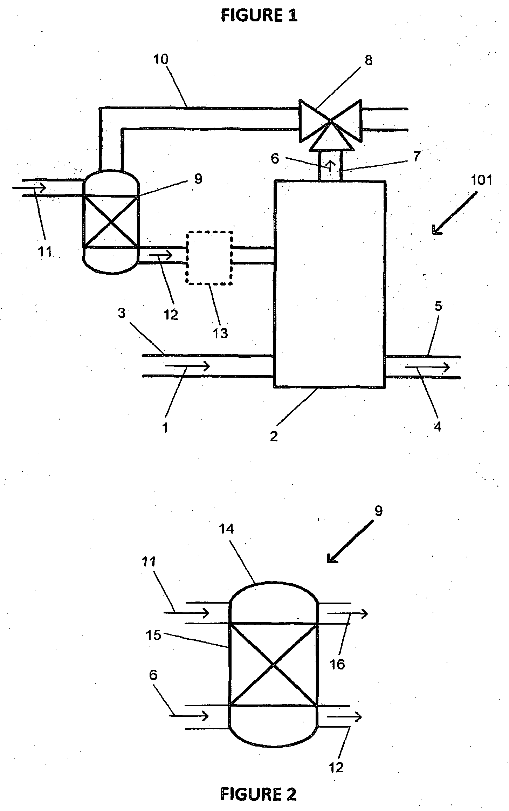

Figure 1 is a schematic representation of a system 101 according to one embodiment of the invention. Substrate stream 1, comprising one or more gaseous components such as CO, is passed to bioreactor 2 via inlet conduit 3. Following contacting with a fermentation broth therein, fermentation broth containing at least a portion of the fermentation products 4 exits the bioreactor 2 via outlet 5, wherein the fermentation broth can be passed to product recovery means (not shown). In addition, a disengaged gaseous outlet stream 6 comprising one or more of unreacted substrate components, gaseous by-product components and/or inert gaseous components, exit bioreactor 2 via outlet conduit 7. Valve 8 is configured to direct at least a portion of outlet stream 6 to deoxygenating means 9 via conduit 10. It will be appreciated by those skilled in the art, any proportion (from a small proportion to substantially all) of the outlet stream 6 may be directed to deoxygenating means 9, depending on the relative sizes of the stream 6 and deoxygenating means 9.

Media 11 is directed into deoxygenating means 9, wherein the media 11 is deoxygenated and at least a portion of one or more products are captured into the media 11, from the outlet stream 6. The deoxygenated media 12 may pass through optional pre-treat 13 before passing to bioreactor 2. Pre-treat 13 may be used to control various aspects of the media, such as temperature, nutrient concentrations and the like. Similarly, the pre-treat can be positioned elsewhere in the system and control other aspects as required.

In particular embodiments, the media 11 passed to the deoxygenating means 9 can be recovered.. fermentation broth 4, wherein one or more products are recovered and optionally microorganisms removed prior to passing at least a portion of the media 11 to the deoxygenating means 9. Additional nutrients may be added to the recovered media prior to or after deoxygenating means 9.

Figure 2 is a schematic representation of deoxygenating means 9, comprising a closed vessel 14, wherein media passes into the deoxygenating means 11 via a first inlet port and gaseous outlet stream 6 passes into the deoxygenating means 11 via a second inlet port. The vessel contains packing material 15, configured to promote effective gas/liquid contact and deoxygenating. The deoxygenated media 12, containing at least a portion of one or more products captured from the outlet gas 6 exits the deoxygenating means 9 via a first outlet port and the waste gas 16, exits the deoxygenating means 9 via a second outlet port, where it can be vented.

## Claims

1. A method of improving the efficiency of an anaerobic fermentation, the method including;
a. In a bioreactor containing one or more microorganisms, fermenting one or more gaseous substrates to produce one or more products and a gaseous exit stream;
b. flowing a liquid media broth into a deoxygenating means;
c. passing the gaseous exit stream from the bioreactor to the deoxygenating means;
d. contacting the liquid media broth with an unfermented gaseous exit stream such that one or more components of the gaseous exit stream are captured in the liquid media broth to provide a substantially deoxygenated liquid media broth; and
e. passing the substantially deoxygenated liquid media broth to the bioreactor.

2. The method of claim 1, wherein the deoxygenating means comprises a packing material for improving gas/liquid contact.

3. The method of claim 1 or 2, wherein the substrate comprises CO.

4. The method of any one of claims 1 to 3, wherein one of the components of the gaseous exit stream is ethanol and wherein at least a portion of the ethanol in the gaseous exit stream is captured in the deoxygenated liquid media broth.

5. The method of any one of claims 1 to 4, wherein one of the components of the gaseous exit stream is H2S, and wherein at least a portion of the H2S is captured in the deoxygenated liquid media broth.

6. The method of any one of claims 1 to 5, wherein the one of more products include acetate and ethanol.

7. A method for improving the efficiency of a fermentation, the method comprising;
a. in a bioreactor comprising one or more microorganisms, fermenting a gaseous substrate comprising CO to produce one or more liquid products and a gaseous exit stream comprising at least ethanol;
b. passing the gaseous exit stream comprising at least ethanol to a deoxygenating means containing a liquid nutrient broth;
c. contacting the gaseous exit stream comprising at least ethanol with the liquid media broth to provide a deoxygenated liquid media broth;
d. capturing at least a portion of the ethanol component of the gaseous exit stream in the deoxygenated liquid media broth; and
e. passing the deoxygenated liquid nutrient media to the bioreactor for fermentation.

8. A method according to any one of claims 1 to 7 wherein the gaseous substrate is derived from an industrial waste gas.

## Patentansprüche

1. Verfahren zur Verbesserung der Effizienz einer anaeroben Fermentation, wobei das Verfahren einschließt:
a. in einem Bioreaktor, welcher einen oder mehrere Mikroorganismus/Mikroorganismen beinhaltet, Fermentieren eines gasförmigen Substrats oder mehrerer gasförmiger Substrate, um ein oder mehrere Produkt(e) und einen gasförmigen Ausstrom herzustellen;
b. Fließen einer Flüssigmedium-Nährlösung in ein sauerstoffentziehendes Mittel;
c. Leiten des gasförmigen Ausstroms von dem Bioreaktor zu dem sauerstoffentziehenden Mittel;
d. Inkontaktbringen der Flüssigmedium-Nährlösung mit einem unfermentierten gasförmigen Ausstrom derart, dass eine oder mehrere Komponente(n) des gasförmigen Ausstroms in der Flüssigmedium-Nährlösung aufgenommen werden, um eine im Wesentlichen desoxigenierte Flüssigmedium-Nährlösung bereitzustellen; und
e. Leiten der im Wesentlichen desoxigenierten Flüssigmedium-Nährlösung zu dem Bioreaktor.

2. Verfahren gemäß Anspruch 1, wobei das sauerstoffentziehende Mittel ein Packungsmaterial für die Verbesserung des Gas/Flüssigkeitskontakts umfasst.

3. Verfahren gemäß Anspruch 1 oder 2, wobei das Substrat CO umfasst.

4. Verfahren gemäß einem der Ansprüche 1 bis 3, wobei eine der Komponenten des gasförmigen Ausstroms Ethanol ist und wobei mindestens ein Teil des Ethanols in dem gasförmigen Ausstrom in der desoxigenierten Flüssigmedium-Nährlösung aufgenommen wird.

5. Verfahren gemäß einem der Ansprüche 1 bis 4, wobei eine der Komponenten des gasförmigen Ausstroms H₂S ist und wobei mindestens ein Teil des H₂S in der desoxigenierten Flüssigmedium-Nährlösung aufgenommen wird.

6. Verfahren gemäß einem der Ansprüche 1 bis 5, wobei das eine oder die mehreren Produkt(e) Acetat und Ethanol einschließen.

7. Verfahren zur Verbesserung der Effizienz einer Fermentation, wobei das Verfahren umfasst:
a. in einem Bioreaktor, welcher einen oder mehrere Mikroorganismus/Mikroorganismen umfasst, Fermentieren eines gasförmigen Substrats, welches CO umfasst, um ein oder mehrere flüssige Produkt(e) und einen gasförmigen Ausstrom herzustellen, welcher mindestens Ethanol umfasst;
b. Leiten des gasförmigen Ausstroms, welcher mindestens Ethanol umfasst, zu einem sauerstoffentziehenden Mittel, welches eine flüssige Nährlösung beinhaltet;
c. Inkontaktbringen des gasförmigen Ausstroms, welcher mindestens Ethanol umfasst, mit der Flüssigmedium-Nährlösung, um eine desoxigenierte Flüssigmedium-Nährlösung bereitzustellen;
d. Aufnehmen von mindestens einen Teil der Ethanolkomponente aus dem gasförmigen Ausstrom in die desoxigenierte Flüssigmedium-Nährlösung; und
e. Leiten des desoxigenierten flüssigen Nährmediums zu dem Bioreaktor zur Fermentierung.

8. Verfahren gemäß einem der Ansprüche 1 bis 7, wobei das gasförmige Substrat aus einem industriellen Abgas stammt.

## Revendications

1. Procédé pour améliorer l'efficacité d'une fermentation anaérobie, le procédé comportant :
a. dans un bioréacteur contenant un ou plusieurs microorganisme(s), la fermentation d'un ou de plusieurs substrat(s) gazeux pour produire un ou plusieurs produit(s) et un flux de sortie gazeux ;
b. l'introduction d'un bouillon d'agents liquides dans un moyen de désoxygénation ;
c. le transfert du flux de sortie gazeux du bioréacteur au moyen de désoxygénation ;
d. la mise du bouillon d'agents liquides au contact d'un flux de sortie gazeux non fermenté de telle sorte qu'un ou plusieurs constituant(s) du flux de sortie gazeux soit/soient retenus dans le bouillon d'agents liquides afin de réaliser un bouillon d'agents liquides sensiblement désoxygéné ; et
e. le transfert du bouillon d'agents liquides sensiblement désoxygéné dans le bioréacteur.

2. Procédé selon la revendication 1, dans lequel le moyen de désoxygénation comprend un matériau de garnissage pour améliorer le contact gaz / liquide.

3. Procédé selon la revendication 1 ou 2, dans lequel le substrat comprend du CO.

4. Procédé selon l'une quelconque des revendications 1 à 3, dans lequel un des constituants du flux de sortie gazeux est de l'éthanol et dans lequel au moins une partie de l'éthanol du flux de sortie gazeux est retenue dans le bouillon d'agents liquides désoxygéné.

5. Procédé selon l'une quelconque des revendications 1 à 4, dans lequel un des constituants du flux de sortie gazeux est de l'H₂S et dans lequel au moins une partie de l'H₂S du flux de sortie gazeux est retenu dans le bouillon d'agents liquides désoxygéné.

6. Procédé selon l'une quelconque des revendications 1 à 5, dans lequel le/les produit(s) comprend/comprennent de l'acétate et de l'éthanol.

7. Procédé pour améliorer l'efficacité d'une fermentation anaérobie, le procédé comportant :
a. dans un bioréacteur contenant un ou plusieurs microorganisme(s), la fermentation d'un substrat gazeux contenant du CO pour produire un ou plusieurs produit(s) et un flux de sortie gazeux contenant au moins de l'éthanol ;
b. le transfert du flux de sortie gazeux, contenant au moins de l'éthanol, dans un moyen de désoxygénation contenant un bouillon de nutriments liquides ;
c. la mise du flux de sortie gazeux contenant au moins de l'éthanol au contact du bouillon d'agents liquides afin de réaliser un bouillon d'agents liquides sensiblement désoxygéné ;
d. la retenue, dans le bouillon d'agents liquides sensiblement désoxygéné, d'au moins une partie du constituant sous forme d'éthanol du flux de sortie gazeux ; et
e. le transfert, pour fermentation dans le réacteur, du bouillon de nutriments liquides sensiblement désoxygéné.

8. Procédé selon l'une quelconque des revendications 1 à 7, dans lequel le substrat gazeux est dérivé d'un gaz résiduaire industriel.
